# EUROPÄISCHE PATENTSCHRIFT

(11) **EP 0 967 959 B1**
(45) Veröffentlichungstag und Bekanntmachung des Hinweises auf die Patenterteilung: **19.11.2003**
(21) Anmeldenummer: 98916820.8
(22) Anmeldetag: 03.03.1998
(51) Int. Cl.: A61K 7/48, A61K 7/06

(54) **KOSMETISCHES KÖRPERPFLEGEPRÄPARAT UND VERFAHREN ZU SEINER HERSTELLUNG**
COSMETIC BODY-CARE PREPARATION AND PROCESS FOR PREPARING THE SAME
PREPARATION COSMETIQUE DE SOINS CORPORELS ET PROCEDE DE FABRICATION

(30) Priorität: 03.03.1997 DE 19708478
(43) Veröffentlichungstag der Anmeldung: 05.01.2000
(73) Patentinhaber: Strathausen, Gerold, 84079 Bruckberg (DE)
(72) Erfinder: Strathausen, Gerold, 84079 Bruckberg (DE)
(74) Vertreter: Pausch, Thomas, Dipl.-Phys.
(86) Internationale Anmeldenummer: DE9800605
(87) Internationale Veröffentlichungsnummer: WO98038968

(56) Entgegenhaltungen:
- DE-A- 2 612 508
- FR-A- 2 582 218
- US-A- 5 407 675
- CHEMICAL ABSTRACTS, vol. 88, no. 6, 6. Februar 1978 Columbus, Ohio, US; abstract no. 41555v, S. YAMAMOTO: "chlorella extracts and honey for skin cosmetics" XP002074633 & JP 52 125 635 A (CHLORELLA SUN CO., LTD) 21. Oktober 1977
- CHEMICAL ABSTRACTS, vol. 118, no. 22, 31. Mai 1993 Columbus, Ohio, US; abstract no. 219499, T. KIBE: "manufacture of cosmetic and pharmaceutical creams containing honey and natural fat/oil" XP002075056 & JP 05 043 473 A (KEN HOORII KENKYUSCHO KK) 23. Februar 1993
- ENCYCLOPEDIA OF COMMON NATURAL INGREDIENTS, XP002074632
- DATABASE WPI Week 9303 Derwent Publications Ltd., London, GB; AN 93-024974 XP002074634 "Preparation of massaging lotion-containing arnica tincture and oils of chrysanthemae-, champhora-, -carvone pine and peppermint, calendula extract in ethanol" & RO 101 669 A (NIVEA INTR PROD COSMETICE) 30. Juli 1991

## Beschreibung

Die Erfindung betrifft ein kosmetisches Körperpflegepräparat, insbesondere als Kosmetikzubereitung zum Baden, Duschen und/oder zur Haarpflege oder Haarwäsche und ein Verfahren zu seiner Herstellung.

Dieses kosmetische Körperpflegepräparat soll als Körpervollbad, Körperteilbad, als Duschbad und/oder als Haarpflege- oder Haarwäschemittel angewandt werden.

Herkömmliche Badezusätze oder ähnliche Körperpflegepräparate enthalten oft die verschiedensten chemischen Zusätze, welche nicht immer unbedenklich sind. Sie können z. B. Allergien, Ekzeme oder Entzündungen hervorrufen. Säurewidrige Substanzen, z. B. Seifen, können den natürlichen Säureschutzmantel der Haut angreifen oder gar zerstören.

Aus der US-A-5407675 ist ein Pflegemittel für die Kopfhaut und den Augenbrauenbereich auf der Grundlage getrockneter Nesselblätter bekannt geworden, wobei das Mittel einen Honigzusatz aufweist.

Aus der FR-A-2582218 ist eine Haarpflegecreme mit einem Honiganteil von 25 % bekannt geworden.

Aus Chemical Abstract, Band 88, Nr. 6, 6. Februar 1978, Columbus, Ohio, US; Zusammenfassung Nr. 41555v (JP-A-52125635) ist ein Hautpflegemittel auf der Grundlage von Chlorella-Extrakten mit einem Honiganteil von 20 bis 30 % bekannt geworden.

Aus Chemical Abstract, Band 118, Nr. 22, 31. Mai 1993, Columbus, Ohio, US; Zusammenfassung Nr. 219499 (JP-A-05043473) ist ein weiteres kosmetisches Pflegemittel mit einem Squalane-Anteil von 20 % und einem Honiganteil von 80 % bekannt geworden.

Der Erfindung liegt die Aufgabe zugrunde, ein kosmetisches Körperpflegepräparat, insbesondere als Kosmetikzubereitung zum Baden, Duschen und/oder zur Haarpflege oder Haarwäsche und ein Verfahren zu seiner Herstellung bereitzustellen, welches im wesentlichen nur natürliche Bestandteile enthält, hingegen keinerlei chemische Konservierungsstoffe, Emulgatoren, Bindemittel, Parfümstoffe oder andere chemische Hilfsstoffe enthält, und welches unter Normalbedingungen ohne zeitliche Begrenzung lagerstabil ist, sich also nicht entmischt.

Diese Aufgabe wird bezüglich des kosmetischen Körperpflegepräparates mit den Merkmalen des Anspruches 1 und bezüglich seiner Herstellung mit denen des Anspruches 5 gelöst.

Das erfindungsgemäße kosmetische Körperpflegepräparat, insbesondere als Kosmetikzubereitung zum Baden, Duschen und/oder zur Haarpflege oder Haarwäsche, zeichnet sich dadurch aus, dass es Honig in einem Anteil von 40 bis 60 % des Gesamtgewichtes, insbesondere 50 % des Gesamtgewichtes enthält.

Die Lösung der vorgenanten technischen Problemstellung ist erst nach langwierigen und umfangreichen Versuchsreihen gelungen.

Honig, insbesondere von Waldmischhonig in seinen verschiedenen in Mitteleuropa und anderen Ländern vorkommenden Zusammensetzungen hat eine antibakterielle und keimhemmende, mithin an also heilende, pflegende und schützende Wirkung. Diese ist an sich seit langem bekannt. Wenn auch diese positiven Wirkungen des Honigs noch in großer Verdünnung nachweisbar sind, so tritt eine spürbare Wirkung erst bei einer hohen Dosierung ein. Im Handel befindliche Badezusätze enthalten aber nur bis zu 2 % Honig. Es werden auch Badezusätze mit ätherischen Ölen oder Kräuterextrakten angeboten, weil auch deren gesundheitsfördernde Wirkungen bekannt sind. Darüberhinaus sind auch Tenside als Waschmittel oder Badezusätze bekannt.

Ein Körperpflegepräparat der erfindungsgemäßen Art, welches Honig, insbesondere Waldhonig oder Waldmischhonig in hoher Konzentration, und darüber hinaus natürliche ätherische Öle und Kräuterextrakte enthält und diese in einem waschaktiven Tensid vereinigt, gibt es bislang weltweit jedoch noch nicht. Der Grund hierfür liegt nicht zuletzt in der außerordentlich schlechten Mischbarkeit von ätherischen Ölen und Kräuterextrakten, insbesondere alkoholischen Kräuterextrakten. Die Mischbarkeit von Honig und Tensiden oder von Tensiden und ätherischen Ölen ist weniger problematisch. Schwieriger ist es wiederum, die letztgenannten Stoffe mit ätherischen Ölen und Kräuterextrakten zu mischen. Nach galenischen Gesichtspunkten ist eine Entmischung der einzelnen Komponenten mit Sicherheit zu erwarten, so dass in der Fachwelt auch ein gewisses Vorurteil gegen ein derartiges kosmetisches Körperpflegepräparat vorhanden sein dürfte.

Mit der Erfindung gelingt es, ein Honig-Kräuter-Körperpflegepräparat bereitzustellen, das nur natürliche Bestandteile enthält. Irgendwelche Konservierungsstoffe, Emulgatoren, Bindemittel, Parfümstoffe oder andere chemische Hilfsstoffe oder auch nur Wasser, sind als Zusätze nicht erforderlich. Das neue Honig-Kräuter-Körperpflegepräparat ist unter Normalbedingungen ohne zeitliche Begrenzung lagerstabil und entmischt sich also nicht.

Nach einem bevorzugten Grundrezept der Erfindung gemäß Anspruch 1 enthält das Körperpflegepräparat die folgenden Bestandteile in Gewichts-%:
Honig 40 mindestens %
Tenside 40 mindestens %
Naturreine ätherische Öle 1 bis 5 %
Alkoholische Kräuterextrakte 0,1 bis 1 %

Bei einem bevorzugten Körperpflegepräparat stellt das Tensid Natriumlaurylaethersulfat dar. Der Anteil der Tenside oder des Tensids kann vorteilhafterweise etwa 48 % betragen.

Zwechmässigerweise beträgt der Anteil der naturreinen ätherischen Öle etwa 1,7 %. Dabei können die naturreinen ätherischen Öle aus einer Mischung insbesondere wie folgt zusammengesetzt sein:
Oleum Rosmarini 28 bis 31 %
Oleum Lavendulae 22 bis 25 %
Oleum Melissae 22 bis 25 %
Oleum Pini pum. 22 bis 25 %
(zusammen 100 %).

Der Anteil der naturreinen ätherischen Öle beträgt somit an der Gesamtzusammensetzung:
Oleum Rosmarini 0,5 %
Oleum Lavendulae 0,4 %
Oleum Melissae 0,4 %
Oleum Pini pum. 0,4 %.

Weiterhin kann bei der besonders bevorzugten Ausführung der Anteil der alkoholischen Kräuterextrakte 0,3 % betragen. Die alkoholischen Kräuterextrakte können bevorzugt aus der folgenden Mischung zusammengesetzt sein:
Extractum Arnicae 32 bis 35 %
Extractum Calendulae mindestens 64 %
(zusammen 100 %)

Der Anteil der alkoholischen Kräuterextrakte beträgt somit an der Gesamtzusammensetzung:
Extractum Arnicae 0,1 %
Extractum Calendulae 0,2 %.

Im weiteren werden die vorteilhaften Eigenschaften der Grundbestandteile beschrieben:

### Der Honig

Es ist besonders vorteilhaft, wenn der Honig ein Waldmischhonig einheimischer oder mitteleuropäischer Nadelhölzer ist, der auch Akazienhonig enthält. Dieser Honig ist Träger zahlreicher nährender, pflegender und heilbringender Inhaltsstoffe und dient zugleich als Konservierungsstoff.

So hat Waldmischhonig von allen Honigsorten den höchsten Anteil an Inhibinen. Das sind Stoffe, die das Wachstum von Bakterien hemmen. Der Gehalt an organischen Säuren des Honigs ist fast identisch mit dem Säuregehalt der menschlichen Haut. Hierdurch ist ein optimaler Schutz des Säureschutzmantels der menschlichen Haut gegeben. Weiterhin sind die Vitamine C, B1, B2, B6, E, Folsäure, Biotin-H und Nikotinsäure PP enthalten. An Spurenelementen sind Mg, SiO₂, P, S, Mn, Si, K, Na, Ca, Cu und Fe vorhanden. Der erfindungsgemäß einzusetzende Honig enthält auch eine Reihe von Aminosäuren, nämlich Leucin, Asparaginsäure, Glutaminsäure, Phenylalanin, Threonin, Arginin, Histidin, Glycin, Lysin, Serin, Valin, Cystin und Prolin. Neben dem natürlichen Wachstumshormon Acetylcholin kommen im Honig noch über 50 natürliche Duftstoffe vor, darunter Isobutyl- und Acetaldehyd, Aceton und Diacetyl.

### Die naturreinen ätherischen Öle

Im Vergleich zu handelsüblichen Badezusätzen enthält das kosmetische Körperpflegepräparat einen sehr hohen Anteil an natürlichen ätherischen Ölen. Diese duften natürlich frisch, verflüchtigen sich und gelangen so über die Atemwege in den Körper. So erzielt man einen angenehmen Körperduft und schafft gleichzeitig eine gesundheitsfördernde Aromatherapie. Das Oleum Rosmarini (Rosmarinöl) wirkt herz-kreislauffördernd und vitalisierend. Das Oleum Lavendulae (Lavendelöl) hat krampflösende, bruhigende, stärkende und desinfizierende Eigen. Das Oleum Melissae (Melissenöl) wirkt ebenfalls beruhigend und entspannend, streßabbauend und ausgleichend. Ebenfalls entspannend wirkt das Oleum Pinipum. (Latschenkieferöl). Dieses lindert aber auch Atemwegs- und Erkältungskrankheiten.

### Die alkoholischen Kräuterextrakte

Als alkoholische Kräuterextrakte werden Extractum Arnicae e flor. fluid. (30 %) 1:1 (Arnikablütenextrakt) und Extractum Calendulae fluid. (35 %) 1:2 (Ringelblumenblütenextrakt) eingesetzt. Diese Extrakte beruhigen und entspannen die durch dauernde Umwelteinflüsse geschädigte Oberhaut in hervorragender Weise. Das Bindegewebe wird gestärkt, die Durchblutung gefördert und Entzündungen gelindert, mitunter auch beseitigt.

### Die Tenside

Unter den Tensiden ist Natriumlaurylaethersulfat eine besonders sinnvolle und milde Waschsubstanz. Es ist aber auch der Einsatz eines anderen ähnlichen milden Tensids möglich. Der Schmutz, der täglich auf die Haut einwirkt und die Stoffwechselprodukte des Organs Haut müssen möglichst schonend entfernt werden, um den wirksamen Inhaltsstoffen des kosmetischen Körperpflegepräparates den Zugang zur Hautoberfläche zu gewährleisten.

Auf Grund all dieser positiven Eigenschaften des erfindungsgemäßen kosmetischen Körperpflegepräparates bietet seine Anwendung einen vorzüglichen Schutz der Haut vor den schädlichen Einflüssen durch Umweltverschmutzung. Es bietet eine deutlich spürbare Linderung der hierdurch hervorgerufenen Hautschädigungen. Auch schon bei Säuglingen und Kindern kann der Kosmetisches Körperpflegepräparat angewandt werden. Seine medizinische Anwendung empfiehlt sich bei folgenden Hauterkrankungen:
- Schuppenbildung der Haut, besonders der Kopfhaut.
- Kopfekzeme, Körperekzeme, besonders bakterieller Art.
- Akne, unterstützende Behandlung bei -eurodermitis, Psoriasis, Flechten und Juckreiz.
- Entzündungen der Schleimhäute besonders im Anal- und Genitalbereich.

Bei einer besonders bevorzugten Zusammensetzung des erfindungsgemäßen kosmetischen Körperpflegepräparates besteht dieses (in Gew.-%) aus:

| | |
|---|---|
| Waldmischhonig | 50,0 % |
| Natriumlaurylaethersulfat | 48,0 % |
| Oleum Rosmarini | 0,5 % |
| Oleum Lavendulae | 0,4 % |
| Oleum Melissae | 0,4 % |
| Oleum Pini pum. | 0,4 % |
| Extractum Arnicae | 0,1 % |
| Extractum Calendulae | 0,2 % |
| | (zusammen 100,0 %). |

Es wird nun die Herstellung einer 10-kg-Charge eines solchen kosmetischen Körperpflegepräparates beschrieben. Selbstverständlich können auch größere Chargen hergestellt werden, sofern die apparativen Voraussetzungen erfüllt sind.

Zunächst werden 5 kg Waldmischhonig schonend im Wasserbad oder in einem ähnlich wirkenden Wärmetauscher auf 30 bis 31° C erwärmt. Beim Erwärmen sollten an der Gefäßwandung des Honiggefäßes auf keinen Fall Temperaturen über 40° C auftreten, weil sonst die Qualität des Honigs in unzulässiger Weise leiden würde. Danach läßt man den Honig langsam auf die Raumtemperatur von 21 bis 22° C abkühlen oder kühlt ihn auf diese Temperatur ab, falls die Raumtemperatur nach unten oder ober abweichen sollte. Sodann wird das Rührwerk in Betrieb genommen. Dabei kann es sich um ein herkömmliches Rührwerk handeln, wie es z. B. von Heimwerkern zum Mischen von Dispersionsfarben verwendet wird. Die Rührerdrehzahl wird auf 2000 bis 2400 min⁻¹ eingestellt.

Nun werden die naturreinen ätherischen Öle einzeln und nacheinander in der Reihenfolge
40 g Oleum Pini pum.,
40 g Oleum Lavendulae,
40 g Oleum Melissae, und
50 g Oleum Rosmarini
zugegeben. Jedes Oleum wird ca. 2 bis 5 min lang eingerührt oder eingemischt, ehe das nächste folgt.

Die alkoholischen Extrakte, nämlich 10 g Extractum Arnicae und 20 g Exträktum Calendulae werden getrennt zunächst 2 bis 4 min lang gemischt und dann in die Mischung aus Waldmischhonig und ätherischen Ölen eingerührt oder eingemischt.

In diese Mischung werden zuletzt 4,8 kg Natriumlaurylaethersulfat eingerührt oder eingemischt. Diese Mischung ist von ihrer Zusammensetzung her das fertige kosmetische Körperpflegepräparat. Dieses muß dann weiterhin bei 20 bis 21° C lichtgeschützt und abgedeckt, aber nicht verschlossen, ca. 30 bis 40 Stunden lang ruhen. Auf diese Weise kann möglicherweise eingeschlagene Luft entweichen.

Als letzter Schritt folgt die Abfüllung des Honig-Kräuter-Körperpflegepräparates in handelsübliche Gefäße, bei der hier geschilderten 10-kg-Charge von Hand, ansonsten in einer herkömmlichen Abfüllanlage.

## Patentansprüche

1. Kosmetisches Körperpflegepräparat, insbesondere als Kosmetikzubereitung zum Baden, Duschen und/oder zur Haarpflege oder Haarwäsche,
**dadurch gekennzeichnet, dass**
es in Gewichts-% mindestens 40 % Honig, mindestens 40 % Tenside, 1 bis 5 % naturreine ätherische Öle, und etwa 0,1 bis 1 % alkoholische Kräuterextrakte (zusammen 100 %) enthält.

2. Körperpflegepräparat nach Anspruch 1, **dadurch gekennzeichnet, dass** die naturreinen ätherischen Öle aus der folgenden Mischung zusammengesetzt sind:
Oleum Rosmarini 28 bis 31 %
Oleum Lavendulae 22 bis 25 %
Oleum Melissae 22 bis 25 %
Oleum Pini pum. 22 bis 25 %
(zusammen 100 %).

3. Körperpflegepräparat nach Anspruch 1 oder 2, **dadurch gekennzeichnet, dass** die alkoholischen Kräuterextrakte aus der folgenden Mischung zusammengesetzt sind:
Extractum Arnicae 32 bis 35 %
Extractum Calendulae mindestens 64 %
(zusammen 100 %).

4. Körperpflegepräparat nach einem oder mehreren der vorhergehenden Ansprüche, **dadurch gekennzeichnet, dass** der Honig Waldhonig ist, der Waldhonig insbesondere auch Akazienhonig enthält, und der Waldhonig insbesondere ein Waldmischhonig einheimischer oder mitteleuropäischer Nadelhölzer ist.

5. Verfahren zur Herstellung eines kosmetischen Körperpflegepräparates, insbesondere als Kosmetikzubereitung zum Baden, Duschen und/oder zur Haarpflege oder Haarwäsche,
**dadurch gekennzeichnet, dass**
es mit Honig zu einem Anteil von mindestens 40 % des Gesamtgewichtes, mindestens 40 % Tenside, 1 bis 5 % naturreine ätherische Öle, und etwa 0,1 bis 1 % alkoholische Kräuterextrakte (zusammen 100 %) gefertigt wird.

6. Verfahren nach Anspruch 5, **gekennzeichnet durch** folgende Verfahrensschritte:
(a) der Honig wird im Wasserbad oder in einem ähnlich wirkenden Wärmetauscher schonend auf 30 bis 31° C erwärmt und anschließend auf 21 bis 22° C langsam abkühlen gelassen oder abgekühlt; bei dieser Temperatur erfolgen alle weiteren Verfahrensschritte; alle Verfahrensschritte folgen unmittelbar aufeinander.
(b) die naturreinen ätherischen Öle werden einzeln und nacheinander zugegeben; jedes Oleum wird ca. 2 bis 5 min lang eingerührt oder eingemischt, ehe das nächste folgt;
(c) die alkoholischen Extrakte werden ca. 2 bis 4 min lang gemischt;
(d) die Mischung der alkoholischen Extrakte nach (c) wird langsam in die Mischung aus Honig und ätherischen Ölen nach (b) eingerührt oder eingemischt;
(e) sodann wird das Tensid eingerührt oder eingemischt.

7. Verfahren nach Anspruch 6, **dadurch gekennzeichnet, dass** das auf diese weise hergestellte Körperpflegepräparat lichtgeschützt bei einer gleichmäßigen Temperatur von ca. 20 bis 21° C abgedeckt, aber nicht verschlossen wird, und 30 bis 40 Stunden ruht, damit die möglicherweise eingeschlagene Luft entweichen kann.

8. Verfahren nach Anspruch 6 oder 7, **dadurch gekennzeichnet, dass** die naturreinen ätherischen Öle einzeln und in der Reihenfolge
Oleum Pini pum.,
Oleum Lavendulae,
Oleum Melissae,
Oleum Rosmarini
zugegeben werden.

## Claims

1. Cosmetic body-care preparation, especially for use as a cosmetic preparation for bath, shower and/or for hair care, **characterized in that** it contains (in weight-%) at least 40 % Honey, at least 40 % Tenside, 1 to 5 % pure natural essential oils, and about 0,1 to 1 % alcoholic herb extracts (total 100 %).

2. Body-care preparation according to claim 1, **characterized in that** the pure natural essential oils are made from the following mixture:
Oleum Rosemary 28 to 31 %
Oleum Lavender 22 to 25 %
Oleum Melissa 22 to 25 %
Oleum Pine 22 to 25 %
(total: 100 %).

3. Body-care preparation according to claim 1 or 2, **characterized in that** the alcoholic herb extracts are made from the following mixture:
Arnica extracts 32 to 35 %
Calendulae extracts at least 64 %
(total 100 %).

4. Body-care preparation according to one or more of the preceding claims, **characterized in that**, the honey is wild honey, the wild honey also contains Acacia honey, and the wild honey is a wild honey mixture indigenous to a native or middle European pine tree.

5. The manufactoring process of a cosmetic body-care preparation, especially as a cosmetic preparation for bath, showering and/or hair care, **characterized in that** it is made with honey with a percentage of at least 40 % of the total weight, at least 40 % tenside, 1 to 5 % pure natural essential oils, and about 0,1 to 1 % alcoholic herb extracts (total 100%).

6. Process according to claim 5 **characterized in** the following processing steps:
(a) the honey is warmed in a water bath or in a similar acting mild heat exchanger to 30° to 31°C and then allowed to cool or cooled slowly to 21° to 22°C; at these temperatures all additional processing steps are carried out; all processing steps are carried out directly one after another;
(b) the pure natural essential oils are added individually and one after the other; each oil is stirred or mixed in for 2 to 5 minutes before the next is added;
(c) the alcoholic extracts are mixed approx. 2 minutes to 4 minutes long;
(d) the alcoholic extract mixture according to step (c) is slowly stirred into or mixed into the mixture of honey and essential oils according to step (b);
(e) thereafter the tenside is stirred or mixed in.

7. Process according to claim 6, **characterized in that** the body-care preparation manufactured in this way, must be covered protected from light but not sealed at an even temperature of 20° to 21°C and lies for 30 to 40 hours thus allowing for any pocketing air to escape.

8. Process according to claim 6 or 7, **characterized in that**, the pure natural essential oils are added one after the other in the following series
Oleum Pine,
Oleum Lavender,
Oleum Melissa,
Oleum Rosemary.

## Revendications

1. Préparation cosmétique de soins corporels, speciallement pour usage comme une preparation cosmetique pour baigner, doucher et/ou pour soins de la chevelure, characterisée en ce qu'elle contient (en % en poids) au moins 40 % de miel, au moins 40 % des tensides, 1 à 5 % des pur naturelles huiles essentielles, et environ 0,1 à 1 % des extraires herbales alcooliques (en totàle 100 %).

2. Préparation de soins corporels selon la revendication 1, characterisée en ce que les huiles pur naturelles essentielles sont composées de la mixture suivante:
Oleum Romarin 28 à 31 %
Oleum Lavende 22 à 25 %
Oleum Mélisse 22 à 25 %
Oleum Pin 22 à 25 %
(en totale: 100 %).

3. Préparation de soins corporels selon la revendication 1 ou 2, characterisée en ce que les extraires herbales alcooliques sont composès de la mixture suivante:
Extraires d'Arnica 32 à 35 %
Extraires de Calendulae au moins de 64 %
(en totale 100 %).

4. Préparation de soins corporels selon d'une ou de plusieures de les revendications précédées, characterisées en ce que le miel est le miel de bois, le miel de bois aussi contient le miel d'acacia, et le miel de bois est une mixture des miels de bois des conifères indigènes ou de l'Europe centrale.

5. Procède de fabrication d'une préparation cosmétique de soins corporels, speciallement pour usage comme une preparation cosmetique pour baigner, doucher et/ou pour soins de la chevelure, characterisée en ce qu'elle est fabriquée avec de miel avec une portion au moins de 40 % de la poids, au moins de 40 % des tensides, 1 à 5 % des pur naturelles huiles essentielles, et environ 0,1 à 1 % des extraires herbales alcooliques (total 100%).

6. Procède selon la revendication 5, characterisée en les pas suivants:
(a) le miel est échauffè avec ménagement dans un bain-marie ou dans une échangeur de chaleur opérant analoguement à 30° à 31°C et après cela lentement laissé rafraîchir ou rafraîchit à 21° à 22°C; à ca temperature tous les autres pas de la procède avont lieu; tous les pas de la procède avont lieu directémment l'un après l'autre;
(b) les pur naturelles huiles essentielles sont ajoutés chacun en particulier et l'un après l'autre; chaque huile est délayé ou mêlé 2 à 5 minutes devant la prochaine est ajouté;
(c) les extraires herbales alcooliques sont mêlès environ 2 minutes à 4 minutes;
(d) la mixture de les extraires alcooliques selon le pas (c) est lentement délayée dans ou mêlèe dans la mixture du miel et des huiles essentielles selon pas (b);
(e) après cela, les tensides sont délayés our mêles.

7. Procède selon la revendication 6, characterisée en ce qu'on devra couvrir la préparation de soins corporel, ce qu'elle est fabriquée de cette manière, protégée de la lumière mais pas cachetée à un temperature unifome de 20° à 21°C et repose pour 30 à 40 heures pour laisser l'air s'échapper, quelle est peut-etre inclus.

8. Procède selon la revendication 6 ou 7, characterisée en ce que les pur naturelles huiles essentielles sont ajoutées l'une après l'autre dans série suivante :
Oleum Pin,
Oleum Lavende
Oleum Mélisse
Oleum Romarin
